# EUROPÄISCHE PATENTSCHRIFT

(11) **EP 1 078 615 B1**
(45) Veröffentlichungstag und Bekanntmachung des Hinweises auf die Patenterteilung: **16.10.2002**
(21) Anmeldenummer: 99890268.8
(22) Anmeldetag: 19.08.1999
(51) Int. Cl.: A61F 11/08

(54) **Gehörschutzvorrichtung**
Hearing protector
Dispositif de protection auditive

(43) Veröffentlichungstag der Anmeldung: 28.02.2001
(73) Patentinhaber: Kurer, Walter, 5610 Wohlen (CH)
(72) Erfinder: Ribic, Zlatan, Dipl.-Ing.Dr., 1232 Wien (AT); Schiess, Hans-Rudolf, 5621 Zufikon (CH)
(74) Vertreter: Babeluk, Michael, Dipl.-Ing. Mag.

(56) Entgegenhaltungen:
- WO-A-91/11160
- AU-A- 5 606 773
- DE-C- 389 521
- DE-U- 29 808 467
- FR-A- 2 558 055
- NL-C- 1 007 937
- US-A- 2 327 620
- US-A- 2 717 596
- US-A- 2 881 759

## Beschreibung

Die Erfindung betrifft eine Gehörschutzvorrichtung zur frequenzselektiven Dämpfung von Tönen, mit einem in den äußeren Gehörgang einer Person einführbaren Gehäuse, in dem ein Übertragungsweg für Töne angeordnet ist, der aus mindestens einer Kammer und mindestens einem sich in die Kammer öffnenden Kanal mit einem vorbestimmten Querschnittsverlauf besteht.

Im Zuge steigender Lärmbelastung kommt einem wirksamen Gehörschutz eine immer größer werdende Bedeutung zu. In vielen Fällen ist es dabei jedoch erforderlich, nicht nur einen wirksamen Schutz des menschlichen Gehörs zu gewährleisten, sondern auch die Kommunikationsfähigkeit der Person zu erhalten. Diese Anforderungen können durch Gehörschutzvorrichtungen erfüllt werden, deren Dämpfungsverhalten frequenzabhängig ist. Das Dämpfungsverhalten wird dabei so gewählt, dass die Dämpfung in dem Bereich der für die Sprachverständlichkeit wichtigen Frequenzen gering ist, während die Dämpfung in dem spezifischen Frequenzbereich des Lärms möglichst groß gewählt wird. In vielen Fällen ist der auszublendende Lärm im Tieftonbereich angesiedelt. In einigen Fällen jedoch, wie etwa bei einem Zahnbohrer, handelt es sich um hohe Frequenzen, die gedämpft werden müssen, um das Ohr zu schützen. Bei unzureichendem Schutz treten zunächst vorläufige Hörverluste auf, bei denen sich die Hörschwelle nach einigen Stunden oder Tagen wieder regeneriert. Bei fortdauernder Lärmexposition tritt dann ein permanenter Hörverlust auf, der auch als permanente Schwellenverschiebung (PTS) bezeichnet wird. Im Allgemeinen tritt zunächst ein Hörverlust im Bereich zwischen 3 kHz und 6 kHz auf, in weiterer Folge verbreitet sich der Hörverlust in den Bereich hoher Frequenzen und erst in der letzten Phase auch in den Bereich tiefer Frequenzen. Eine Heilung dieses Hörverlustes im medizinischen Sinn ist nicht möglich. Daher kommt einem optimalen Gehörschutz große Bedeutung zu.

Aus der WO 91/11160 ist eine Gehörschutzvorrichtung bekannt, die ins Ohr eingeführt wird und die einen oder mehrere Helmholtzresonatoren aufweist Bei einem Helmholtzresonator handelt es sich um eine Kombination einer Kammer, die ein Schwingungsvolumen bildet, mit einem sich in die Kammer öffuenden Kanal. Im elektrotechnischen Analogon bildet der Kanal je nach seinem Durchmesser eine Induktivität oder einen Widerstand, während die Kammer einer Kapazität entspricht. In Abhängigkeit von den geometrischen Abmessungen der einzelnen Bauteile kann auf diese Weise eine frequenzselektive Dämpfung erreicht werden. Es hat sich jedoch herausgestellt, dass ein Dämpfungsverhalten, das für eine bestimmte Art der Lärmexposition gut geeignet ist, in anderen Anwendungsfällen unbefriedigende Ergebnisse zeigt.

Aus der DE 42 17 043 A ist eine Gehörschutzvorrichtung bekannt, die ebenfalls in der Art eines Helmholtzresonators ausgebildet ist. Insbesondere soll durch diese Gehörschutzvorrichtung ein Schutz vor impulsförmigen Geräuschen mit steilen Flanken, also etwa einem Knall, erzielt werden. Der in der Gehörschutzvorrichtung vorgesehene Kanal kann jedoch bei Bedarf durch einen geeigneten Stoppel verschlossen werden, wodurch die Vorrichtung von selektiver Dämpfung auf generelle Dämpfung umgeschaltet werden kann. In diesem Fall ist jedoch das Sprachverständnis deutlich verschlechtert.

Verschiedene Dokumente, wie etwa die AU 56067 73 A, die US 2,327,620 A oder die US 2,881,759 A beschreiben Gehörschutzvorrichtungen, bei denen ein Kanal durch ein Ventil oder einen Verschluss geschlossen werden kann, um die Dämpfung entsprechend zu erhöhen. Bei der Lösung entsprechend der AU 56067 73 A erfolgt das Verschließen durch bewegliche Klappen, bei der Lösung entsprechend der US 2,327,620 A durch verdrehbare Stopfen. Dies ermöglicht es, je nach Bedarf eine geringe Dämpfung oder eine starke Dämpfung zu wählen. Eine frequenzselektive Anpassung ist jedoch so nicht möglich, so dass die Wirkung nur für bestimmte Situationen optimal ist und in anderen Situationen oft unbefriedigend ist.

Aufgabe der vorliegenden Erfindung ist es, diese Nachteile zu vermeiden und eine Vorrichtung der oben beschriebenen Art so weiterzubilden, dass eine Anpassung an unterschiedliche Belastungssituation durchgeführt werden kann. Dies bedeutet, dass die Frequenzkennlinie der Dämpfung in Abhängigkeit von der zu erwartenden Lärmexposition veränderbar sein soll, ohne jedoch die Sprachverständlichkeit übermäßig zu beeinträchtigen.

Erfindungsgemäß ist vorgesehen, dass ein bewegliches Element vorgesehen ist, in dem mehrere unterschiedliche Kanäle vorgesehen sind, die in Abhängigkeit von der jeweiligen Stellung von einem feststehenden Gehäuseteil abgedeckt oder freigeben werden.

Wesentlich an der Erfindung ist, dass durch die Kammer und den Kanal ein Helmholtzresonator gebildet wird, dessen geometrische Verhältnisse an den Einsatzzweck angepasst werden können. Es ist jedoch dabei wesentlich, in einem Frequenzbereich von 1 kHz bis 3 kHz eine relativ geringe Dämpfung zu verwirklichen, um die Sprachverständlichkeit zu erhalten. Musiker haben unter Umständen andere Frequenzbereiche, in denen eine geringe Dämpfung erwünscht ist. Die Dämpfung in den übrigen Frequenzbereichen wird durch die Veränderung des Querschnittsverlaufs des Kanals eingestellt. Die Änderung des Querschnittsverlaufs bedeutet, dass der Kanal, der im Allgemeinen einen in Längsrichtung nicht einheitlichen Querschnitt aufweist, so eingestellt werden kann, dass ein möglichst günstiges Dämpfungsverhalten der gesamten Vorrichtung erzielt wird. Wichtig ist dabei, dass des Kanal nicht einfach verschlossen wird, sondern in seinem Querschnittsverlauf verändert wird. Im Allgemeinen hat der Kanal verschiedene Abschnitte mit unterschiedlichen Querschnitten, und die Anpassung an den gewünschten Verlauf der Übertragungsfunktion erfolgt durch die Veränderung der Querschnittsverlaufs, indem beispielsweise die Länge der einzelnen Abschnitte verändert wird.

In einer ersten Gruppe von Ausführungsvarianten ist diese Veränderbarkeit des Querschnittsverlaufs dadurch gegeben, dass mehrere Kanäle vorgesehen sind, von denen jeweils einer aktiviert wird, während die übrigen verschlossen und damit deaktiviert sind.

In einer bevorzugten Ausführungsvariante ist dabei das bewegliche Element als verdrehbare Trommel ausgebildet, in der mehrere Kanäle mit unterschiedlichen Querschnittsverläufen vorgesehen sind, wobei die einzelnen Kanäle in Abhängigkeit von der jeweiligen Stellung von einem feststehenden Gehäuseteil abgedeckt oder freigegeben werden. In jeder Betriebsstellung der beweglichen Trommel wird dabei von einer Gehäusebohrung einer der Kanäle freigegeben, während die übrigen abgedeckt sind. Zur Verwirklichung besonders spezifischer Frequenzverläufe können theoretisch auch jeweils zwei oder mehrere Kanäle gleichzeitig freigegeben werden und somit parallel geschaltet sein.

Eine besonders platzsparende Ausführung ist gegeben, wenn die Trommel innerhalb der Kammer angeordnet ist.

In einer alternativen Ausführungsvariante der Erfindung kann vorgesehen sein, dass mehrere parallele Kanäle vorgesehen sind, die von dem beweglichen Element abgedeckt oder freigegeben werden. Dadurch kann in manchen Fällen eine konstruktive Vereinfachung erzielt werden.

Vorzugsweise ist vorgesehen, dass mehrere parallele Kanäle vorgesehen sind, die von dem beweglichen Element abgedeckt oder freigegeben werden.

In einer weiteren bevorzugten Ausführungsvariante der Erfindung ist eine stufenlose Veränderbarkeit des Querschnittsverlaufs oder der Länge des Kanals vorgesehen. Dabei wird im Gegensatz zu den obigen Ausführungsvarianten nicht diskret zwischen einzelnen Kanälen umgeschaltet, sondern der Querschnittsverlauf kontinuierlich verändert. Auf diese Weise ist eine besonders feine Justierung möglich.

Eine besonders hohe Dämpfungswirkung wird dann erreicht, wenn im Gebrauchszustand die Kammer über einen ersten Kanal mit dem Gehörgang der Person verbunden ist und über einen weiteren Kanal mit der Umgebung verbunden ist.

Eine hohe Wirksamkeit verbunden mit der Möglichkeit einer kostengünstigen Herstellung kann dadurch erzielt werden, dass der Kanal aus drei Abschnitten besteht, die hintereinander angeordnet sind, wobei der mittlere Abschnitt einen kleineren Querschnitt als die übrigen Abschnitte aufweist. Insbesondere ist es dabei günstig, wenn mehrere parallele Kanäle vorgesehen sind, die sich im Wesentlichen durch die Länge des mittleren Abschnitts unterscheiden.

Die oben beschriebenen Ausführungsvarianten sind primär dazu geeignet, eine regelbare Dämpfung im Tieftonbereich zu verwirklichen. Eine besonders wirksame Regelung im Hochtonbereich ist dann möglich, wenn in der Kammer quer zum Übertragungsweg eine Membran vorgesehen ist. Eine geeignete Membran lässt Schall tiefer Frequenzen mit einer relativ konstanten Dämpfung durch, während im Bereich um die Resonanzfrequenz die Dämpfung weitgehend regelbar ist.

In der Folge wird die vorliegende Erfindung anhand der in den Figuren dargestellten Ausführungsbeispielen näher erläutert. Es zeigen: die
- Fig. 1: schematisch eine allgemeine Gehörschutzvorrichtung, von der die Erfindung ausgeht;
- Fig. 2: ein Schaltungsdiagramm, das das Verhalten einer solchen Gehörschutzvorrichtung beschreibt;
- Fig. 3: eine schematische Darstellung zur Erklärung der Wirkungsweise einer Gehörschutzvorrichtung; die Fig. 4 ein Schaltungsdiagramm zur Fig. 3;
- Fig. 5: ein Diagramm, das das Dämpfungsverhalten einer Vorrichtung nach Fig. 3 zeigt;
- Fig. 6: eine weitere schematische Darstellung einer anderen Ausführungsvariante;
- Fig. 7: ein Schaltungsdiagramm zur Fig. 6;
- Fig. 8: ein Diagramm entsprechend der Fig. 5 für die Ausführungsvariante nach Fig. 6;
- Fig. 9: einen Längsschnitt durch eine Ausführungsvariante der vorliegenden Erfindung;
- Fig. 10: eine axonometrische Explosionsdarstellung der Ausführungsvariante von Fig. 9;
- Fig. 11: einen Schnitt durch eine Trommel der Ausführungsvariante von Fig. 9;
- Fig. 11A, 11B, 11C, 11D und 11E: Schnitte entsprechend den Linien A-A, B-B, C-C, D-D bzw. E-E in Fig. 11;
- Fig. 12: einen Schnitt durch eine weitere Ausführungsvariante der Erfindung;
- Fig. 13: eine axonometrische Explosionsdarstellung der Ausführungsvariante von Fig. 12;
- Fig. 14: eine weitere Ausführungsvariante der Erfindung im Schnitt;

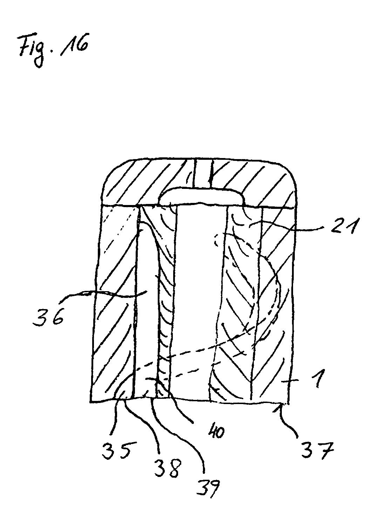

- Fig. 15: eine Stirnansicht der Ausführungsvariante von Fig. 14 und
- Fig. 16: eine teilweise Darstellung einer weiteren Ausführungsvariante im Schnitt.

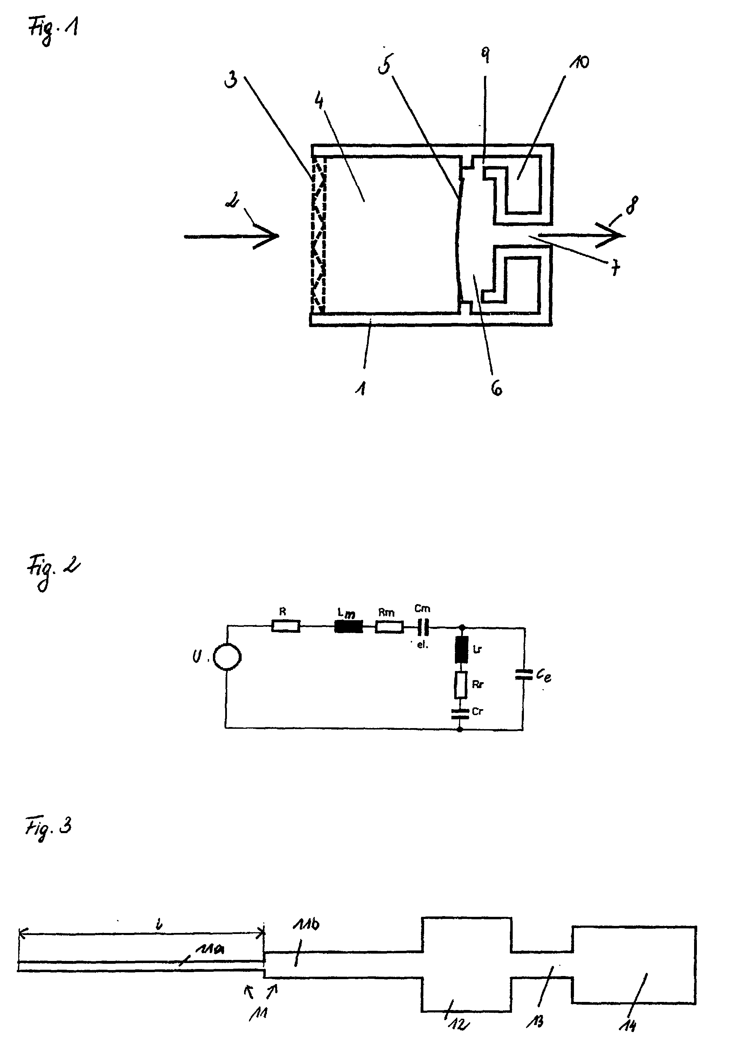

In der Fig. 1 ist der allgemeine Aufbau einer Gehörschutzvorrichtung nach dem Stand der Technik gezeigt. Ein Gehäuse 1 ist in seiner Form entweder so aufgebaut, dass die Vorrichtung in den äußeren Gehörgang eingeführt werden kann, oder es wird das Gehäuse in einen entsprechenden Passteil, der hier nicht dargestellt ist, eingebaut, um eine optimale Anpassung an die jeweiligen anatomischen Verhältnisse zu erzielen. Die entsprechend dem Pfeil 2 ankommenden Schallwellen treten durch ein Netz 3 in eine erste Kammer 4 ein. Die Kammer 4 ist durch eine Membran 5 abgeschlossen, und die Schallwellen können über eine weitere Kammer 6 und eine Öffnung 7 entsprechend dem Pfeil 8 die Vorrichtung ins Innere des Ohres verlassen. Mit der weiteren Kammer 6 ist über Kanäle 9 ein Saugvolumen 10 verbunden, das das Dämpfungsverhalten der Vorrichtung beeinflusst.

In der Fig. 2 ist ein elektrisches Ersatzschaltbild für die Vorrichtung von Fig. 1 gezeigt, das das Verhalten dieser Vorrichtung beschreibt. Die Schallwelle entspricht einer Wechselspannungsquelle U, die an einer Kapazität Cₑ anliegt, die das Ohr darstellt. In Serie zu der Kapazität Cₑ ist ein erster Widerstand R geschaltet, der die akustische Impedanz des Netzes 3 abbildet. Weiters sind eine Induktivität Lₘ, ein Widerstand Rₘ und eine Kapazität Cₘ in Serie geschaltet, die die Membran 5 abbilden. Die Masse der Membran ist dabei verantwortlich für die Induktivität, während der Widerstand Rₘ die in der Membran 5 verursachte Reibung abbildet. Die Kapazität Cₘ spiegelt die Elastizität der Membran 5 wider.

Weiters sind parallel zu der Kapazität Cₑ des Ohres eine weitere Induktivität Lᵣ, ein weiterer Widerstand Rᵣ und eine weitere Kapazität Cᵣ geschaltet. Dies entspricht den Kanälen 9 und dem Saugvolumen 10.

Es ist einleuchtend, dass durch eine entsprechende Wahl der geometrischen Verhältnisse der Vorrichtung von Fig. 1 und der Art der Masse und der Spannung der Membran 5 das Dämpfungsverhalten der Vorrichtung in Abhängigkeit von der Frequenz stark beeinflusst werden kann. So ist es beispielsweise möglich, ein weitgehend gleichmäßiges Dämpfungsverhalten über einen großen Frequenzbereich einzustellen. Dieses Dämpfungsverhalten ist jedoch nicht veränderlich.

In der Fig. 3 ist schematisch der grundsätzliche Aufbau einer Ausführungsvariante der vorliegenden Erfindung dargestellt, die im Wesentlichen als Tiefpassfilter ausgebildet ist, bei dem die Dämpfung im unteren Frequenzbereich veränderlich ist. In diesem Fall sind hintereinander vorgesehen: ein Kanal 11, bestehend aus einem ersten Kanalabschnitt 11a mit kleinem Querschnitt und einem zweiten Kanalabschnitt 11b mit größerem Querschnitt, ein Volumen 12, ein weiterer Kanal 13 und ein Volumen 14, das das Ohr darstellt. Es hat sich gezeigt, dass ein Kanal mit einem kleinen Querschnitt akustisch einem Widerstand entspricht, während ein Kanal mit größerem Querschnitt eher einer Induktivität entspricht. Dies hat seinen Grund darin, dass bei Kanälen mit kleinem Querschnitt die Reibung dominiert, während bei etwas größeren Querschnitten Schwingungsvorgänge der Luft eine Rolle spielen. In dem interessanten Frequenzbereich kann man von einem Querschnitt von etwa 0,5 mm bis 0,8 mm Durchmesser ausgehen, unterhalb dem ein Widerstandsverhalten zu beobachten ist, während oberhalb die Induktivität dominiert.

Das Wesen der Erfindung besteht nun darin, die Länge der Kanalabschnitte 11a und 11b zu verändern, um das Verhalten der Gehörschutzvorrichtung optimal an die jeweiligen Bedürfnisse anpassen zu können.

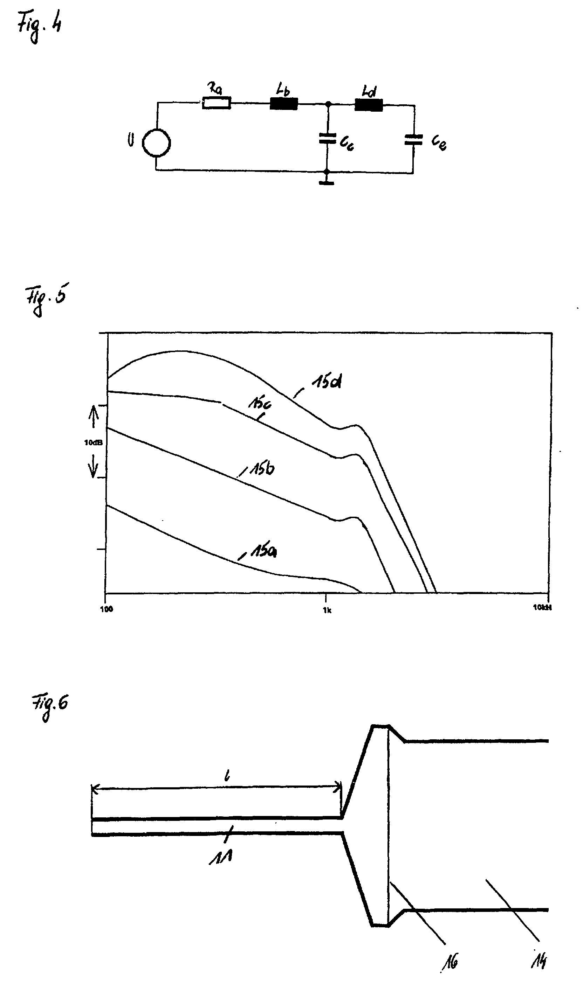

In der Fig. 4 ist das entsprechende Schaltungsdiagramm zu Fig. 3 dargestellt. Wiederum entspricht U der Schallquelle, der Widerstand Rₐ entspricht dem ersten Kanalabschnitt 11a, die Induktivität dem zweiten Kanalabschnitt 11b, die Kapazität C_{c} dem Volumen 11, und die weitere Induktivität dem weiteren Kanal 13. Cₑ stellt wiederum das Ohr 14 dar.

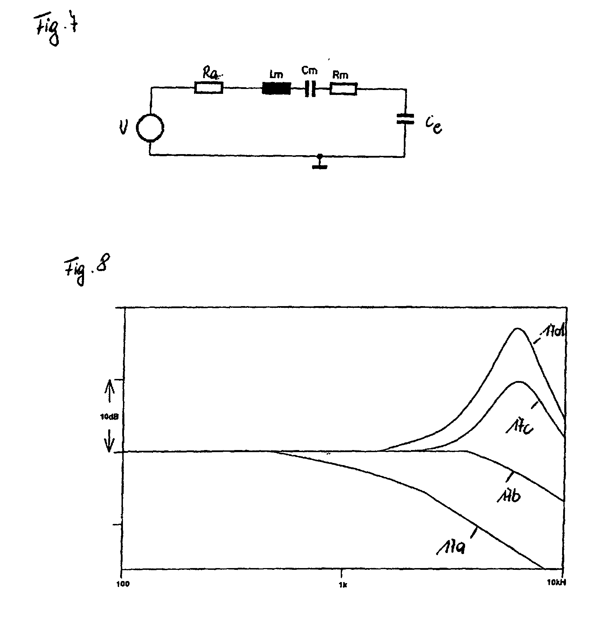

In der Fig. 5 ist schematisch ein Dämpfungsdiagramm gezeigt, bei dem der Ausgangsschallpegel über der Frequenz aufgetragen ist. In der Fig. 5 sind vier Kurven 15a, 15b, 15c, 15d dargestellt, die das Übertragungsverhalten der Anordnung von Fig. 3 in Abhängigkeit von der Frequenz darstellen. Die Kurve 15a entspricht dabei einer Anordnung in Fig. 3 mit einer großen Länge 1 des ersten Kanalabschnitts 11a. Die Kurve 15b entspricht einer Anordnung gemäß Fig. 3 mit kleinerem 1, während die Kurven 15c und 15d einem noch kürzeren ersten Kanalabschnitt 11a entsprechen.

Die Fig. 6 zeigt schematisch eine andere Ausführungsvariante der Erfindung, die als ein im Hochtonbereich regelbares Filter ausgebildet ist, mit einem Kanal 11, einer Membran 16 und einem Ohrvolumen 14. In der Fig. 7 ist das analoge Schaltbild dargestellt, wobei Rₐ wiederum dem Widerstand des Kanals 11 entspricht und die Induktivität Lₘ, die Kapazität Cₘ und der Widerstand Rₘ das elektrische Analogon der Membran 16 darstellen. In der Fig. 8 sind wiederum Kurven 17a, 17b, 17c und 17d eingezeichnet, die dem Übertragungsverhalten der Anordnung von Fig. 6 mit unterschiedlicher Länge 1 des Kanals 11 entsprechen. Die Kurve 17a entspricht dabei dem Kanal 11 mit der größten Länge 1, während die Kurve 17d dem Kanal 11 mit der geringsten Länge 1 entspricht.

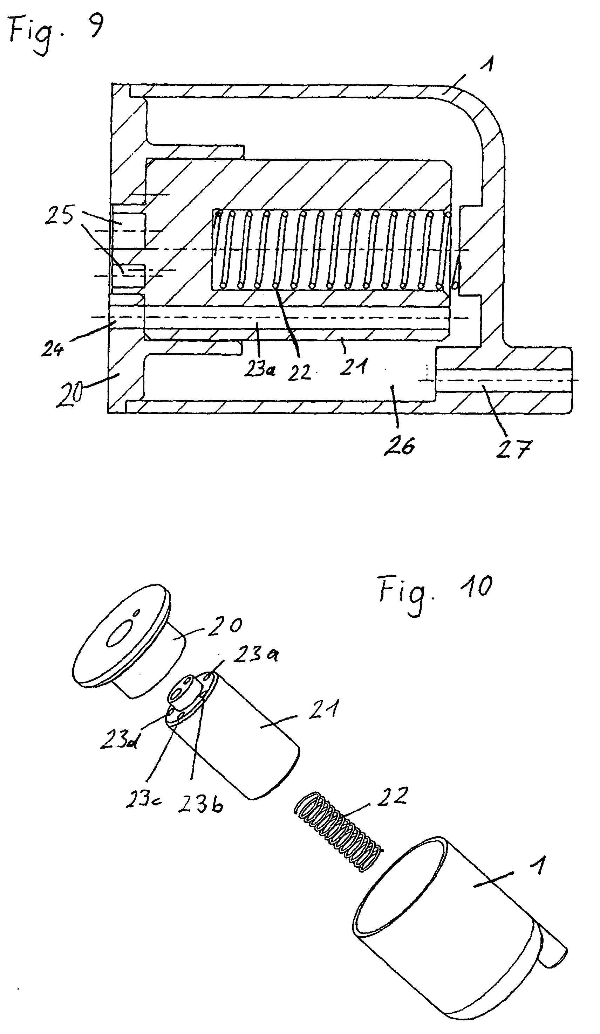

In der Fig. 9 ist der konstruktive Aufbau einer ersten Ausführungsvariante der Erfindung dargestellt. Ein Gehäuse 1 ist mit einem Deckel 20 verschlossen. In dem Gehäuse 1 ist weiters eine Trommel 21 drehbar geführt, die von einer Feder 22 gegen den Deckel 20 gedrückt wird. In der Trommel 21 sind in Axialrichtung mehrere Bohrungen angeordnet, von denen eine, die den Kanal 23a bildet, in der Fig. 9 ersichtlich ist. Eine Bohrung 24 im Deckel 20 verbindet den Kanal 23 mit der Umgebung. Weitere Bohrungen 25 in der Stirnseite der Trommel 21 dienen dazu, mit einem entsprechenden nicht dargestellten Werkzeug die Trommel 21 zu verdrehen. Der Raum innerhalb des Gehäuses 1 und um die Trommel 21 bildet eine Kammer 26, in die sich der Kanal 23a öffnet. Von der Kammer 26 führt ein weiterer Kanal 27, der sich im Gebrauchszustand der Gehörschutzvorrichtung in den äußeren Gehörgang öffnet.

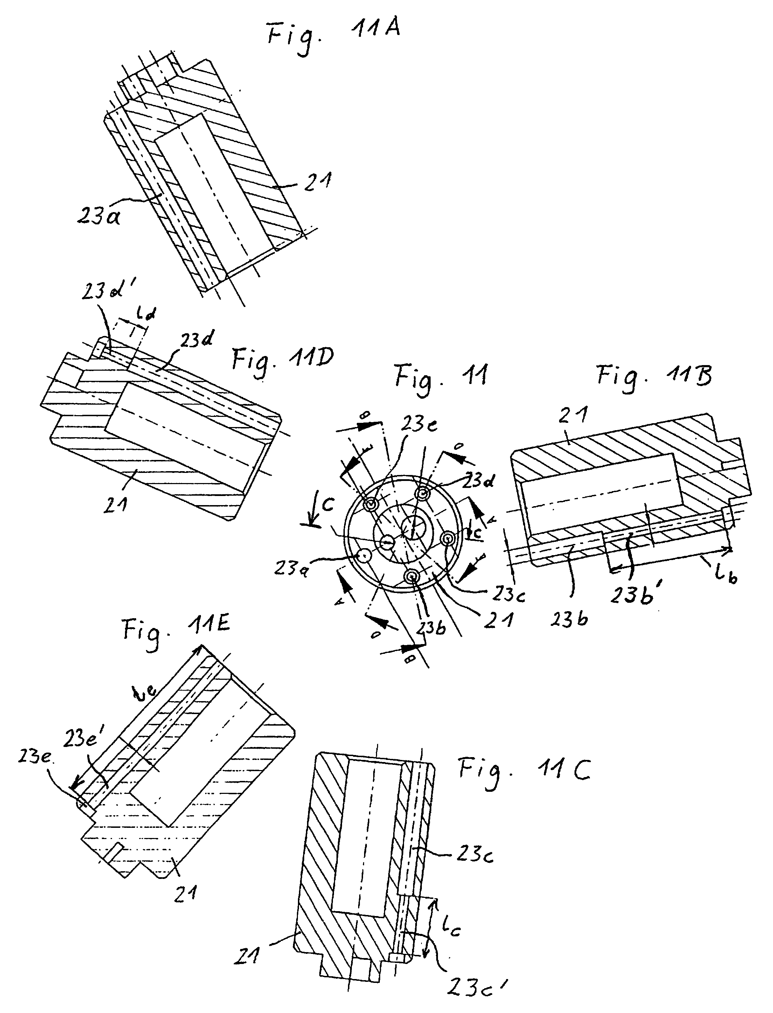

In der Fig. 10 ist in einer Explosionsdarstellung der Aufbau der Vorrichtung von Fig. 9 in verkleinertem Maßstab dargestellt. Es ist ersichtlich, dass parallel zu dem Kanal 23a weitere Kanäle 23b, 23c, 23d sowie ein weiterer hier nicht ersichtlicher Kanal ausgebildet sind.

In den Fig. 11 sowie in den Fig. 11A, 11B, 11C, 11D und 11E ist die genaue Ausbildung der Trommel 21 ersichtlich. Der Kanal 23a in Fig. 11a ist durchgängig mit einem vergleichsweise großen Durchmesser von beispielsweise 1,5 mm ausgeführt. Der dazu parallele Kanal 23b besitzt einen mittleren Kanalabschnitt 23b' mit einem kleinen Durchmesser von beispielsweise 0,3 mm. Die Länge dieses Kanalabschnitts 23b ist 1_{b}. Die Kanäle 23c und 23d sind ähnlich aufgebaut wie der Kanal 23b, unterschiedlich ist nur, dass der mittlere Kanalabschnitt 23c' bzw. 23d' mit verringertem Querschnitt eine unterschiedliche Länge 1_{c} bzw. 1_{d} aufweist. Der Kanal 23e aus Fig. 11E besitzt einen Kanalabschnitt 23e', dessen Länge 1ₑ nahezu der Gesamtlänge des Kanals 23e entspricht.

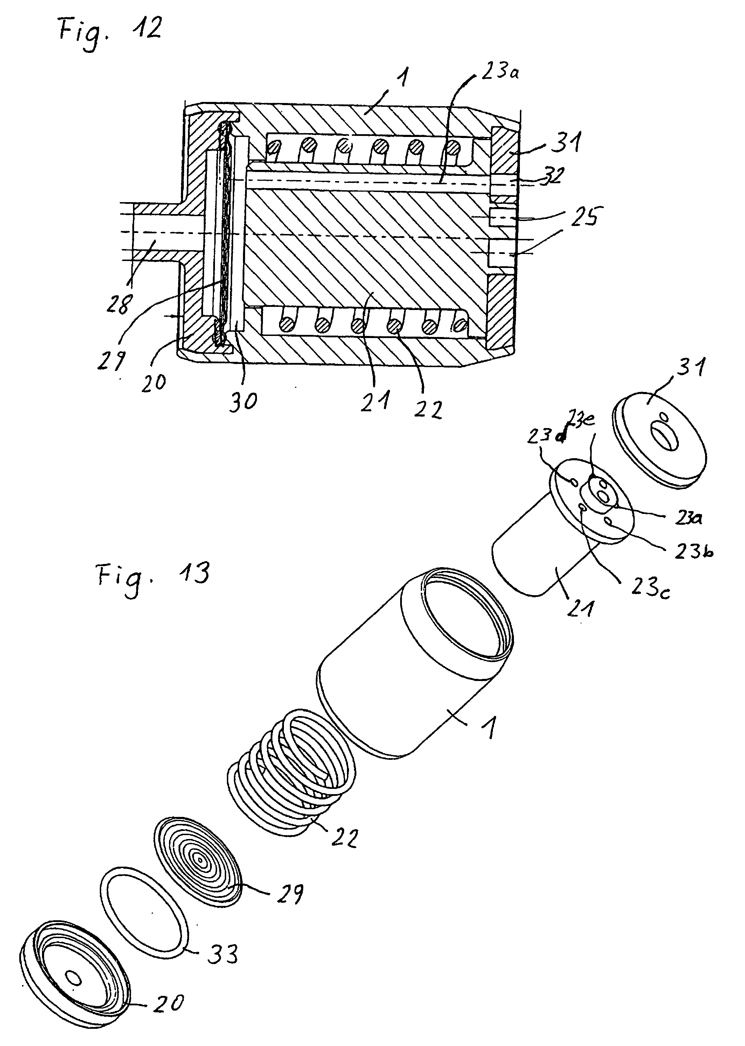

In der Fig. 12 ist eine weitere Ausführungsvariante der Erfindung in einem Längsschnitt dargestellt. Eine Öffnung 28 dient zum Austritt des Schalls, der eine Membran 29 durchdrungen hat, die in einer Kammer 30 quer zur Ausbreitungsrichtung des Schalls angeordnet ist. Stromaufwärts davon ist eine Trommel 21 vorgesehen, die analog der Trommel der Fig. 9 bis 11 ausgebildet ist, mit dem Unterschied, dass die Feder 22 außerhalb der Trommel 21 angeordnet ist. Auch in der Trommel 21 der Fig. 12 sind fünf parallele Kanäle 23a bis 23e angeordnet, wie aus der Explosionsdarstellung von Fig. 13 ersichtlich ist. Durch eine Öffnung 32 in einem weiteren Deckel 31 treten die Schallwellen in die erfindungsgemäße Vorrichtung ein. Die Membran 29 ist gegenüber dem Gehäuse 1 und dem Deckel 20 durch einen O-Ring 33 abgedichtet und festgeklemmt.

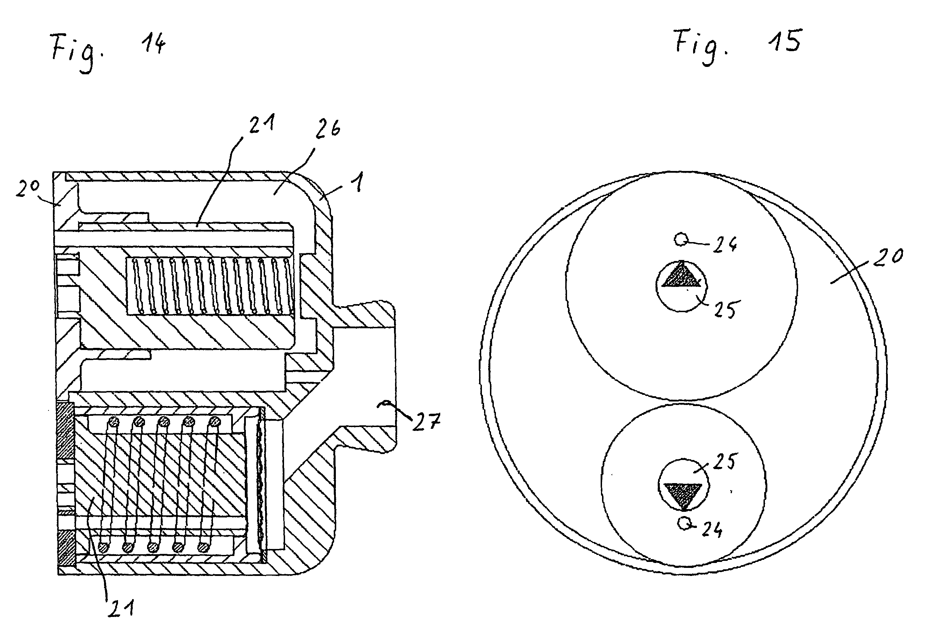

In der Fig. 14 ist eine Ausführungsvariante der Erfindung dargestellt, bei der in einem Gehäuse die Ausführungsvarianten der Fig. 9 bis 11 und der Fig. 12 und 13 miteinander kombiniert und parallel zueinander angeordnet sind. Eine detaillierte Beschreibung des Aufbaus ist daher nicht weiter erforderlich. Da die beiden Trommeln 21 unabhängig voneinander verstellt werden können, ergibt sich eine weitgehend unabhängige Regelungsmöglichkeit im Hochton- bzw. im Tieftonbereich. Dies ermöglicht eine optimale Anpassung an schwierige Verhältnisse. Falls eine gleichzeitige forcierte Dämpfung sowohl im Tiefton- als auch im extremen Hochtonbereich gefordert wird, kann auch statt einer Parallelschaltung eine Serienschaltung von zwei oder mehreren Einzelvorrichtungen vorgenommen werden.

In der Fig. 16 ist schematisch eine Ausführungsvariante gezeigt, bei der eine stufenlose Verstellmöglichkeit gegeben ist. Dabei ist in dem Gehäuse 1 eine im Wesentlichen schraubenförmig ausgebildete Nut 35 eingefräst. Die Trommel 21, die in dem Gehäuse 1 verdrehbar geführt ist, besitzt eine einzige sich in Axialrichtung erstreckende Nut 36 an ihrem äußeren Umfang. Der Schall kann über eine Eintrittsöffnung 38 in die schraubenförmige Nut 35 eintreten und über eine Öffnung 39 aus der Nut 36 der Trommel 21 austreten. Beide Öffnungen 38 und 39 sind in der Stirnfläche 37 des Gehäuses 1 bzw. der Trommel 21 angeordnet. Je nach der Drehstellung der Trommel 21 verschiebt sich der Überscheidungspunkt 40 der Nuten 35 und 36 und damit die Länge des Kanals zwischen den Öffnungen 38 und 39. Wie bei den anderen Ausführungsvarianten können die Querschnitte der Kanäle 35 und 36 unterschiedlich ausgeführt sein, um einerseits eine Induktivität und andererseits einen Widerstand zu bilden.

Mit der vorliegenden Erfindung ist es möglich, das Dämpfungsverhalten einer Gehörschutzvorrichtung in Abhängigkeit von den jeweils bestehenden Bedürfnissen gezielt zu verändern und zu optimieren. Die Anpassung kann werksmäßig bei der Herstellung vorgenommen werden, wodurch es möglich ist, mit ein und derselben Vorrichtung eine Vielzahl unterschiedlich wirkender Gehörschutzvorrichtungen herzustellen und anzubieten. Sowohl der Produktionsaufwand als der Aufwand für die Lagerhaltung können dadurch deutlich gesenkt werden. Es ist aber auch möglich, während des Gebrauchs das Dämpfungsverhalten einzustellen, um geänderten Umweltbedingungen Rechnung zu tragen. Damit ist eine besonders flexible Einstellung auf die jeweiligen Geräuschverhältnisse in besonders vorteilhafter Weise möglich.

## Patentansprüche

1. Gehörschutzvorrichtung zur frequenzselektiven Dämpfung von Tönen, mit einem in den äußeren Gehörgang einer Person einführbaren Gehäuse, in dem ein Übertragungsweg für Töne angeordnet ist, der aus mindestens einer Kammer (26) und mindestens einem sich in die Kammer (26) öffnenden Kanal (11; 23a, 23b, 23c, 23d) mit einem vorbestimmten Querschnittsverlauf besteht, **dadurch gekennzeichnet, dass** ein bewegliches Element vorgesehen ist, in dem mehrere unterschiedliche Kanäle (11; 23a, 23b, 23c, 23d) vorgesehen sind, die in Abhängigkeit von der jeweiligen Stellung von einem feststehenden Gehäuseteil abgedeckt oder freigeben werden.

2. Gehörschutzvorrichtung nach Anspruch 1, **dadurch gekennzeichnet, dass** das bewegliche Element als verdrehbare Trommel (21) ausgebildet ist.

3. Gehörschutzvorrichtung nach Anspruch 2, **dadurch gekennzeichnet, dass** die Trommel (21) innerhalb der Kammer angeordnet ist.

4. Gehörschutzvorrichtung nach Anspruch 1, **dadurch gekennzeichnet, dass** die Kanäle parallel sind und von dem beweglichen Element abgedeckt oder freigegeben werden.

5. Gehörschutzvorrichtung nach einem der Ansprüche 1 bis 4, **dadurch gekennzeichnet, dass** die Kanäle (23a, 23b, 23c, 23d) aus drei Abschnitten bestehen, die hintereinander angeordnet sind, wobei der mittlere Abschnitt (23a', 23b') einen kleineren Querschnitt als die übrigen Abschnitte aufweist.

6. Gehörschutzvorrichtung nach Anspruch 5, **dadurch gekennzeichnet, dass** die Kanäle (23a, 23b, 23c, 23d) sich im wesentlichen durch die Länge (I) des mittleren Abschnitts (23a', 23b') unterscheiden.

7. Gehörschutzvorrichtung nach einem der Ansprüche 1 bis 6, **dadurch gekennzeichnet, dass** in der Kammer (30) quer zum Übertragungsweg eine Membran (29) vorgesehen ist.

8. Gehörschutzvorrichtung nach einem der Ansprüche 1 bis 7, **dadurch gekennzeichnet, dass** im Übertragungsweg mehrere Kammern hintereinander oder parallel angeordnet sind.

## Claims

1. A hearing protection device for damping sounds in a frequency-selective manner, comprising a housing which can be inserted into the external auditory canal of a person, in which a transmission path for sounds is disposed, consisting of at least one chamber (26) and at least one channel (11; 23a, 23b, 23c, 23d) with a predetermined cross-sectional progress which opens into said chamber (26), **characterized in that** a movable element is provided in which several different channels (11; 23a, 23b, 23c, 23d) are provided which are covered or released by a fixed housing part depending on the respective position.

2. A hearing-protection device as claimed in claim 1, **characterized in that** the movable element is arranged as a rotatable drum (21).

3. A hearing-protection device as claimed in claim 2, **characterized in that** the drum (21) is arranged within the chamber.

4. A hearing-protection device as claimed in claim 1, **characterized in that** the channels are parallel and are covered or released by the movable element.

5. A hearing-protection device as claimed in one of the claims 1 to 4, **characterized in that** the channels (23a, 23b, 23c, 23d) consist of three sections which are disposed one after the other, with the middle section (23a', 23b') having a smaller cross section than the other sections.

6. A hearing-protection device as claimed in claim 5, **characterized in that** the channels (23a, 23b, 23c, 23d) substantially differ by the length (I) of the middle section (23a', 23b').

7. A hearing-protection device as claimed in one of the claims 1 to 6, **characterized in that** a membrane (29) is provided in the chamber (30) transversally to the transmission path.

8. A hearing-protection device as claimed in one of the claims 1 to 7, **characterized in that** several chambers are disposed in parallel or behind one another in the transmission path.

## Revendications

1. Dispositif de protection acoustique pour amortir les sons de manière sélective en fréquence, avec un boîtier qui peut être introduit dans le conduit auditif externe d'une personne et dans lequel est aménagé un trajet de transfert des sons qui est constitué par au moins une chambre (26) et par au moins un canal (11 ; 23a, 23b, 23c, 23d) s'ouvrant dans la chambre (26) et ayant une section transversale présentant un profil prédéterminé, **caractérisé en ce que** l'on prévoit un élément mobile dans lequel est aménagée une pluralité de différents canaux (11 ; 23a, 23b, 23c, 23d) qui sont couverts ou exposés en fonction de chaque position d'une partie fixe du boîtier.

2. Dispositif de protection acoustique selon la revendication 1, **caractérisé en ce que** l'élément mobile est réalisé sous la forme d'un tambour rotatif (21).

3. Dispositif de protection acoustique selon la revendication 2, **caractérisé en ce que** le tambour (21) est agencé à l'intérieur de la chambre.

4. Dispositif de protection acoustique selon la revendication 1, **caractérisé en ce que** les canaux sont parallèles et qu'ils peuvent être couverts ou exposés grâce à l'élément mobile.

5. Dispositif de protection acoustique selon l'une des revendications 1 à 4, **caractérisé en ce que** les canaux (23a, 23b, 23c, 23d) sont constitués de trois portions disposées en série, la portion médiane (23a', 23b') ayant une section transversale inférieure à celle des autres portions.

6. Dispositif de protection acoustique selon la revendication 5, **caractérisé en ce que** les canaux (23a, 23b, 23c, 23d) sont essentiellement différents par la longueur (I) de la portion médiane (23a', 23b').

7. Dispositif de protection acoustique selon l'une des revendications 1 à 6, **caractérisé en ce que** dans la chambre (30) on prévoit une membrane (29) perpendiculaire au trajet de transmission.

8. Dispositif de protection acoustique selon l'une des revendications 1 à 7, **caractérisé en ce que** dans le trajet de transmission, il y a plusieurs chambres disposées en série ou en parallèle.
